# EUROPEAN PATENT SPECIFICATION

(11) **EP 4 023 968 B1**
(45) Date of publication and mention of the grant of the patent: **02.08.2023**
(21) Application number: 21210132.3
(22) Date of filing: 24.11.2021
(51) Int. Cl.: F25D 17/04, F25D 29/00, G01N 33/00

(54) **A REFRIGERATOR COMPRISING ODOR SENSOR**
KÜHLSCHRANK MIT GERUCHSSENSOR
RÉFRIGÉRATEUR COMPRENANT UN CAPTEUR D'ODEURS

(30) Priority: 31.12.2020 TR 202022619
(43) Date of publication of application: 06.07.2022
(73) Proprietor: Arçelik Anonim Sirketi, 34445 Istanbul (TR)
(72) Inventor: ARSAN, Ismet, 34445 ISTANBUL (TR); CELIK, Aydin, 34445 ISTANBUL (TR); AK, Samet, 34445 ISTANBUL (TR); FADAEI FOULADI, Ramin, 34445 ISTANBUL (TR)

(56) References cited:
- WO-A2-2018/063126
- KR-A- 20200 136 190
- US-A1- 2007 266 725
- US-A1- 2017 262 973
- US-A1- 2020 097 776

## Description

The present invention relates to a refrigerator comprising odor sensors for detecting whether foodstuff placed in a storage compartment thereof is spoiled. The invention also relates to a method of controlling a refrigerator.

Foodstuff stored in a refrigerator spoils in the course of time. Spoilage may not be clearly visible on the surface of certain food. It is then difficult for the user to detect if the spoilage has started. When foodstuff such as meat starts to spoil, it produces various gases such as ammonia. The amount of volatile organic compounds (VOC), that is, odor produced and released by foodstuff in the storage compartment gives an indication of spoilage. By using odor sensors (gas sensors) in the refrigerator, level of VOC and hence spoilage of foodstuff can be detected, and the user can be warned accordingly.

Metal oxide (MOX) sensors are one of the common odor sensor types used for this purpose. A MOX sensor comprises a heater and a sensing material placed on top of the heater. When the sensor is active, the sensing material is heated by the heater. Hydrocarbons of the VOC are adsorbed on the heated sensing material, and in turn change the resistance of the sensing material. By reading the sensor resistance value, therefore, an indication of odor intensity in the environment can be obtained.

When odor sensors are active for an extended time, the increased temperature of the active sensor leads to degradation of the sensing element. As a result, after a certain time of activity odor sensors may deteriorate in terms of sensitivity and may not function properly.

In the state of the art, Chinese Patent Application CN108872486A discloses a refrigerator with gas sensors. The gas sensors are monitored by a monitor for fault information.

Chinese Patent Application CN110285642A discloses a control method for a refrigerator. The refrigerator comprises a plurality of odor sensors for detecting odor therein with an improved accuracy. US 2020/097776 A1 discloses a refrigerator according to the preamble of claim 1. WO 2018/063126 A2, US 2007/266725 A1 and US 2017/262973 A1 disclose refrigerators of the prior art. KR202001360190 discloses a further prior art refrigerator in which two odour sensors are used sequentially to extend the operational life of the odour sensing device.

The aim of the present invention is realization of a refrigerator and a control method thereof, wherein the spoiling process of foodstuff in a refrigerator compartment is reliably detected by odor sensors. Another purpose of the present invention is maintaining the odor detection accuracy in a refrigerator for an extended period of use time. A related purpose of the invention is realization of a refrigerator wherein an operating condition, such as accuracy and sensitivity of an odor sensor is regularly monitored in a facilitated and cost effective way. Another related purpose is realizing a refrigerator that can inform a user about an operating condition of an odor sensor, and hence preventing unnoticed spoilage of foodstuff stored in the refrigerator due to deteriorated sensitivity or malfunctioning of the odor sensor.

Refrigerator realized in order to attain the aim of the present invention, explicated in Claim 1 and the respective claims thereof, comprises at least one storage compartment for storing and refrigerating food; an odor sensing unit comprising a main sensor and at least one auxiliary sensor for sensing odor in the storage compartment, and a processing means for receiving and processing data from the main and auxiliary sensors; and a display for displaying information.

In the invention, the odor sensing unit has a normal operation mode for sensing odor in the storage compartment and a sensitivity check mode for checking sensitivity of the main sensor. The auxiliary sensor is inactive in the normal operation mode and active in the sensitivity check mode. The auxiliary sensor is only occasionally operated as an alternative sensor to cross check the functioning of the main sensor. The total active time of the auxiliary sensor is much shorter than the main sensor, hence the auxiliary sensor is less susceptible to deteriorating due to extended use.

The main sensor and the auxiliary sensor are preferably metal oxide (MOX) sensors reacting in accordance with the level of volatile organic compounds (VOC) in the storage compartment.

The processing means is preferably arranged to, in the sensitivity check mode, compare the reactions of the main sensor and the auxiliary sensor in order to determine a current operating condition of the main sensor. If the difference between reactions of the main sensor and the auxiliary sensor is determined to be greater than a predetermined threshold, the processing means may generate a signal for outputting a warning.

The processing means may be further arranged to automatically activate/deactivate the sensitivity check mode in accordance with a preprogrammed schedule.

According to another aspect of the invention, there is provided a method of controlling a refrigerator. The refrigerator controlled according to the method comprises at least one storage compartment for storing and refrigerating food, an odor sensing unit comprising a main sensor and an at least one auxiliary sensor for sensing odor in the storage compartment, a processing means for receiving and processing data from the main and auxiliary sensors, and a display. The method comprises: operating the odor sensing unit in a normal operation mode wherein the main sensor is active and the auxiliary sensor is inactive; determining whether criteria for switching to a sensitivity check mode is met; if the criteria not met, continuing operating in normal operation mode; if the criteria are met, operating the odor sensing unit in sensitivity check mode wherein both the main sensor and the auxiliary sensor are active; determining whether difference between reactions of the main sensor and the auxiliary sensor are above a predetermined threshold; if not, switching the odor sensing unit back to normal operation mode; and if said difference is above the threshold, generating a warning signal.

The refrigerator and the method of controlling the household appliance realized in order to attain the aim of the present invention is illustrated in the attached figures, where:
Figure 1 is a schematic diagram of a refrigerator according to an embodiment of the invention,
Figure 2 is a flow diagram of the method of controlling the refrigerator according to an embodiment of the invention.

The elements illustrated in the figures are numbered as follows:
1. Refrigerator
2. Odor sensing unit
3. Main sensor
4. Auxiliary sensor
5. Processing means
6. Display

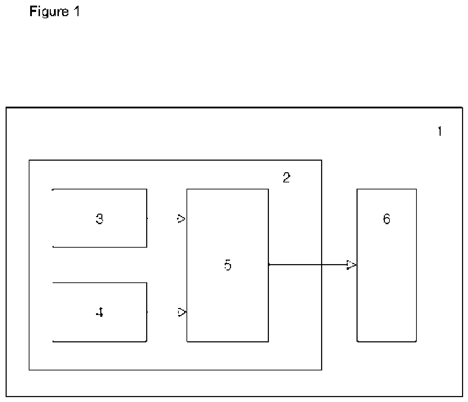

The refrigerator (1) comprises at least one storage compartment for storing and refrigerating food and an odor sensing unit (2). The odor sensing unit (2) comprises a main sensor (3) and one or more auxiliary sensors (4) for sensing odor in the storage compartment, and a processing means (5) for receiving and processing data from the main and auxiliary sensors (3, 4). The processing means (5) may also be arranged to control the operation of the odor sensing unit (2), for example by turning the sensors (3, 4) on/off individually. The refrigerator further comprises a display (6) for displaying information, such as information relating to sensor status. (Figure 1) The display (6) may comprise one or more display units, such as a dedicated display unit for the odor sensing unit (2) and/or a general purpose display unit for the refrigerator (1).

The odor sensing unit (2) has a normal operation mode for sensing odor in the storage compartment and a sensitivity check mode for checking sensitivity of the main sensor (3). The auxiliary sensor (4) is inactive in the normal operation mode and active in the sensitivity check mode. The main sensor (3) is active, that is, at least electrically powered, in both normal operation mode and the sensitivity check mode.

The main sensor (3) and the auxiliary sensor (4) may be metal oxide (MOX) sensors reacting to the level of volatile organic compounds (VOC) in the storage compartment. These sensors (3, 4) typically react to the presence of VOC by a change in their electrical resistance values, by means of which the level of VOC and hence abnormal odor in the storage compartment is detected.

When sensors (3, 4) are active, they are electrically powered and actively detecting the odors in the environment. In case of MOX sensors, being active means the heating element comprised in the sensor is active and the sensing material is heated to high temperatures. On the other hand, when a sensor (3, 4) is inactive; the sensor (3, 4) and a heating element thereof is not powered, and the sensing material is not being heated.

The main sensor (3) and the one or more auxiliary sensors (4) are arranged to sense the VOC level of the same storage compartment in the refrigerator (1). In refrigerators (1) having two or more storage compartments, one main sensor (3) and at least one auxiliary sensor (4) per compartment may be used. The main sensor (3) and the auxiliary sensor (4) working together in a storage compartment are preferably of the same type, and even identical sensors. In order to ensure said pair of sensors (3, 4) are exposed to same odor, they are preferably placed close to one another, for example at a few centimeters, at a maximum of 10 cm distance.

When the odor sensing unit (2) is in sensitivity check mode, the processing means (5) compares the reactions of the main sensor (3) and the auxiliary sensor (4) in order to determine a current operating condition of the main sensor (3). In this manner, possible malfunctioning, operational fault and/or sensitivity loss in the main sensor (3), which may arise due to extended continuous use of the main sensor (3), can be reliably detected by employing the auxiliary sensor (4).

If the difference between reactions of the main sensor (3) and the auxiliary sensor (4) turns out to be greater than a predetermined threshold, it is interpreted as a potential abnormality and a signal for outputting a warning is generated by the processing means (5). Accordingly, a user of the refrigerator (1) can be informed about the abnormality detected by the processing means (5).

The warning signal is transmitted to an output means for outputting a visual, audio and/or a haptic warning to the user. The display (6) comprising one or more light emitting means and/or display screens may be used for this purpose, for example for prompting a visual indication about operating condition, deteriorated functioning and/or sensitivity of the main sensor (3).

The warning may comprise a fault code defining the type of abnormality detected, information indicating that the main sensor (3) should be repaired and/or replaced, etc. Additionally, or alternatively, if the refrigerator (1) is a smart refrigerator capable of wirelessly communicating with external devices, the warning may be transmitted to and outputted by an external mobile device, such as a smartphone or a tablet.

According to an embodiment of the invention, the warning comprises a prompt to operate the odor sensing unit (2) in a backup mode. The backup mode is a further operational mode of the odor sensing unit (2), wherein the main sensor (3) is inactive and the auxiliary sensor is active (4). In order to switch to the backup mode, therefore, the auxiliary sensor (4) is activated and the main sensor (3) is deactivated. The backup mode thus can be considered an operation mode wherein, when compared to the normal operation mode, the auxiliary sensor (4) replaces the main sensor (3) in that the function of the main sensor (3) is overtaken by the auxiliary sensor (4).

The processing means (5) may be arranged to, when the difference between reactions of the main sensor (3) and the auxiliary sensor (4) is greater than the predetermined threshold, automatically switch the sensing unit (2) to the backup mode. In case of detecting abnormality in operation of the main sensor (3), the processing means may also block the operating of the odor sensor unit (2) in normal mode, for example by deactivating the main sensor (3).

In embodiments of the invention, the odor sensing unit (2) can also be manually controlled by an operator or a user, for example by means of a user interface provided on the refrigerator (1) or on an external device in communication with the refrigerator (1). A user can therefore turn the odor sensing unit (2) on/off and select the mode for operating the odor sensing unit (2). When an abnormality is detected during sensitivity check mode, the processing means (5) may block the normal mode of the odor sensing unit (2) by disallowing the user select the normal mode and allowing the odor sensing unit (2) to be operated only in backup mode.

In preferred embodiments of the invention, the processing means (5) is adapted to automatically activate/deactivate the sensitivity check mode in accordance with a preprogrammed schedule. The preprogrammed schedule may comprise a periodic routine. For example, the processing means (5) may be configured to activate the sensitivity check mode on a weekly, biweekly or monthly basis. In this way, operating condition of the main sensor (3) can be monitored regularly and a degrading sensitivity of the main sensor (3) can be detected in a relatively early phase.

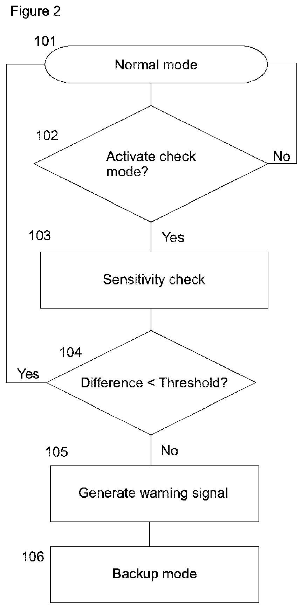

The method of controlling the refrigerator (1) comprises: operating the odor sensing unit (2) in a normal operation mode (101) wherein the main sensor (3) is active and the auxiliary sensor (4) is inactive, and continuously determining whether criteria for switching to a sensitivity check mode is met (102). If the criteria are not met, the odor sensing unit (2) is continued operating in normal operation mode. If the criteria are met, the odor sensing unit (2) switched to a sensitivity check mode (103) wherein both the main sensor (3) and the auxiliary sensor (4) are active. In the sensitivity check mode, it is determined whether difference between reactions of the main sensor (3) and the auxiliary sensor (4) are above a predetermined threshold; if not, the odor sensing unit (2) is switched back to normal operation mode (101). If the difference is above said threshold, a warning signal is generated (105). (Figure 2)

Criteria for switching to a sensitivity check mode may comprise user input indicating selection of sensitivity check mode, a predetermined time count, and/or any command from the processing means (5) triggering sensitivity check mode.

According to an embodiment of the method, if the difference between reactions of the main sensor (3) and the auxiliary sensor (4) is above the predetermined threshold (7), the odor sensing unit (2) is automatically put into a backup mode (106) wherein the main sensor (3) is inactive and the auxiliary sensor (4) is active.

The method may further comprise, after going into the backup mode, checking whether the abnormality detected with the main sensor (3) is eliminated, for example by replacing the defective main sensor (3) with a new one, and switching back to normal operation mode.

At least some, and preferably all steps of the method of controlling the refrigerator (1) are carried out automatically; preferably by the processing means (5).

A major advantageous effect of the present invention is that the sensing of the odor relating to spoilage of foodstuff in a refrigerator (1) is improved by virtue of checking the operating condition of the main sensor (3) by an auxiliary sensor (4), and warning a user of the refrigerator (1) when an abnormality thereof is detected. By occasionally activating an auxiliary sensor (4) in a storage compartment where a main sensor (3) is active for an extended time, the sensing performance of the main sensor (3) is closely monitored. Thus, negative effects of a defective main sensor (3) on refrigerator's (1) food spoilage sensing performance can be eliminated and odors arising from spoiling food can be reliably sensed. Thereby, user satisfaction can be increased. Other advantageous effects of the present invention can be taken from the above described embodiments.

## Claims

1. A refrigerator (1) comprising: at least one storage compartment for storing and refrigerating food; an odor sensing unit (2) comprising a main sensor (3) and an auxiliary sensor (4) for sensing odor in the storage compartment, and a processing means (5) for receiving and processing data from the main and auxiliary sensors (3, 4); and a display (6) for displaying information; **characterized in that** the odor sensing unit (2) has a normal operation mode for sensing odor in the storage compartment and a sensitivity check mode for checking sensitivity of the main sensor (3), wherein the auxiliary sensor (4) is inactive in the normal operation mode and both sensors (3,4) are active in the sensitivity check mode.

2. A refrigerator (1) as in claim 1, wherein the main sensor (3) and the auxiliary sensor (4) are metal oxide (MOX) sensors reacting in accordance with the level of volatile organic compounds (VOC) in the storage compartment.

3. A refrigerator (1) as in claim 1 or 2, wherein the processing means (5) is arranged to, in the sensitivity check mode, compare the reactions of the main sensor (3) and the auxiliary sensor (4) in order to determine a current operating condition of the main sensor (3).

4. A refrigerator (1) as in claim 3, wherein the processing means (5) is further arranged to generate a signal for outputting a warning if the difference between reactions of the main sensor (3) and the auxiliary sensor (4) is greater than a predetermined threshold.

5. A refrigerator (1) as in claim 4, wherein the warning comprises a visual indication on the display (6).

6. A refrigerator (1) as in claim 4 or 5, wherein the warning comprises a prompt to operate the odor sensing unit (2) in a backup mode wherein the main sensor (3) is inactive and the auxiliary sensor is active (4).

7. A refrigerator (1) as in any one of claims 4 to 6, wherein the processing means (5) is further arranged to, if the difference between reactions of the main sensor (3) and the auxiliary sensor (4) is greater than the predetermined threshold, switch the sensing unit (2) to a backup mode wherein the main sensor (3) is inactive and the auxiliary sensor is active (4).

8. A refrigerator (1) as in any one of claims 4 to 7, wherein the processing means (5) is further arranged to, if the difference between reactions of the main sensor (3) and the auxiliary sensor (4) is greater than the predetermined threshold, block the operating of the odor sensor unit (2) in normal mode.

9. A refrigerator (1) as in any one of preceding claims, wherein the processing means (5) is arranged to automatically activate/deactivate the sensitivity check mode in accordance with a preprogrammed schedule.

10. A refrigerator (1) as in claim 9, wherein the preprogrammed schedule comprises a periodic routine.

11. A method of controlling a refrigerator (1) comprising: at least one storage compartment for storing and refrigerating food; an odor sensing unit (2) comprising a main sensor (3) and an auxiliary sensor (4) for sensing odor in the storage compartment, and a processing means (5) for receiving and processing data from the main and auxiliary sensors (3, 4); and a display (6); the method comprising:
operating the odor sensing unit (2) in a normal operation mode (101) wherein the main sensor (3) is active and the auxiliary sensor (4) is inactive, determining whether criteria for switching to a sensitivity check mode is met (102), if the criteria are not met, continuing operating in normal operation mode,
if the criteria are met, operating the odor sensing unit (2) in sensitivity check mode (103) wherein both the main sensor (3) and the auxiliary sensor (4) are active,
determining whether difference between reactions of the main sensor (3) and the auxiliary sensor (4) are above a predetermined threshold; if not, switching the odor sensing unit (2) back to normal operation mode (101), and if said difference is above the threshold, generating a warning signal (105).

12. A method as in claim 11, wherein the method further comprises, if the difference between reactions of the main sensor (3) and the auxiliary sensor (4) is above the predetermined threshold (7), operating the odor sensing unit in a backup mode (106) wherein the main sensor (3) is inactive and the auxiliary sensor (4) is active.

## Patentansprüche

1. Ein Kühlschrank (1) umfasst: mindestens ein Lagerfach zum Aufbewahren und Kühlen von Lebensmitteln; eine Geruchserfassungseinheit (2), die einen Hauptsensor (3) und einen Hilfssensor (4) zum Erkennen von Gerüchen im Aufbewahrungsfach umfasst, und eine Verarbeitungseinrichtung (5) zum Empfangen und Verarbeiten von Daten von den Haupt- und Hilfssensoren (3, 4); und ein Display (6) zum Anzeigen von Informationen; **gekennzeichnet ist es dadurch,** dass die Geruchserfassungseinheit (2) über einen Normalbetriebsmodus zum Erfassen von Gerüchen im Aufbewahrungsfach und einen Empfindlichkeitsprüfmodus zum Überprüfen der Empfindlichkeit des Hauptsensors (3) verfügt, wobei der Hilfssensor (4) im Normalbetriebsmodus inaktiv ist und beide Sensoren (3,4) im Empfindlichkeitsprüfmodus aktiv sind.

2. Ein Kühlschrank (1), wie in Anspruch 1 aufgeführt, wobei der Hauptsensor (3) und der Hilfssensor (4) Metalloxidsensoren (MOX) sind, die entsprechend dem Gehalt an flüchtigen organischen Verbindungen (VOC) im Lagerfach reagieren.

3. Ein Kühlschrank (1), wie in Anspruch 1 oder 2 aufgeführt, wobei die Verarbeitungseinrichtung (5) dazu ausgelegt ist, im Empfindlichkeitsprüfmodus die Reaktionen des Hauptsensors (3) und des Hilfssensors (4) zu vergleichen, um einen aktuellen Betriebszustand des Hauptsensors (3) zu bestimmen.

4. Ein Kühlschrank (1), wie in Anspruch 3 aufgeführt, wobei die Verarbeitungseinrichtung (5) ferner dazu eingerichtet ist, ein Signal zur Ausgabe einer Warnung zu erzeugen, wenn die Differenz zwischen den Reaktionen des Hauptsensors (3) und des Hilfssensors (4) größer als ein vorgegebener Schwellenwert ist.

5. Ein Kühlschrank (1), wie in Anspruch 4 aufgeführt, wobei die Warnung eine visuelle Anzeige auf dem Display (6) umfasst.

6. Ein Kühlschrank (1), wie in Anspruch 4 oder 5 aufgeführt, wobei die Warnung eine Aufforderung umfasst, die Geruchserfassungseinheit (2) in einem Backup-Modus zu betreiben, in dem der Hauptsensor (3) inaktiv und der Hilfssensor aktiv (4) ist.

7. Ein Kühlschrank (1), wie in den Ansprüchen 4 bis 6 aufgeführt, wobei die Verarbeitungseinrichtung (5) außerdem dazu eingerichtet ist, wenn die Differenz zwischen den Reaktionen des Hauptsensors (3) und des Hilfssensors (4) größer als der vorgegebene Schwellenwert ist, um die Sensoreinheit (2) in einen Backup-Modus zu schalten, in dem der Hauptsensor (3) inaktiv und der Hilfssensor aktiv (4) ist.

8. Ein Kühlschrank (1), wie in den Ansprüchen 4 bis 7 aufgeführt, wobei die Verarbeitungseinrichtung (5) außerdem dazu eingerichtet ist, den Betrieb der Geruchssensoreinheit (2) zu blockieren, wenn die Differenz zwischen den Reaktionen des Hauptsensors (3) und des Hilfssensors (4) größer als der vorgegebene Schwellenwert im normalen Modus ist.

9. Ein Kühlschrank (1), wie in einem der vorherigen Ansprüchen aufgeführt, wobei die Verarbeitungseinrichtung (5) so angeordnet ist, dass sie den Empfindlichkeitsprüfmodus gemäß einem vorprogrammierten Zeitplan automatisch aktiviert/deaktiviert.

10. Ein Kühlschrank (1), wie in Anspruch 9 aufgeführt, wobei der vorprogrammierte Zeitplan eine periodische Routine umfasst.

11. Ein Verfahren zur Steuerung eines Kühlschranks (1) umfasst: mindestens ein Lagerfach zum Aufbewahren und Kühlen von Lebensmitteln; eine Geruchserfassungseinheit (2), die einen Hauptsensor (3) und einen Hilfssensor (4) zum Erkennen von Gerüchen im Aufbewahrungsfach umfasst, und eine Verarbeitungseinrichtung (5) zum Empfangen und Verarbeiten von Daten von den Haupt- und Hilfssensoren (3; 4); und ein Display (6); wobei das Verfahren Folgendes umfasst:
Betreiben der Geruchserfassungseinheit (2) in einem normalen Betriebsmodus (101), in dem der Hauptsensor (3) aktiv und der Hilfssensor (4) inaktiv ist,
Bestimmen, ob die Kriterien zum Umschalten in einen Empfindlichkeitsprüfmodus erfüllt sind (102), wenn die Kriterien nicht erfüllt sind, Fortsetzen des Betriebs im normalen Betriebsmodus,
wenn die Kriterien erfüllt sind, Betreiben der Geruchserfassungseinheit (2) im Empfindlichkeitsprüfmodus (103), in dem sowohl der Hauptsensor (3) als auch der Hilfssensor (4) aktiv sind,
Bestimmen, ob die Differenz zwischen den Reaktionen des Hauptsensors (3) und des Hilfssensors (4) über einem vorgegebenen Schwellenwert liegt; wenn nicht, Zurückschalten der Geruchserfassungseinheit (2) in den normalen Betriebsmodus (101), und
wenn die Differenz über dem Schwellenwert liegt, Erzeugen eines Warnsignals (105).

12. Ein Verfahren nach Anspruch 11, wobei das Verfahren des Weiteren folgendes umfasst, wenn die Differenz zwischen den Reaktionen des Hauptsensors (3) und des Hilfssensors (4) über dem vorgegebenen Schwellenwert (7) liegt, Betreiben der Geruchserfassungseinheit in einem Backup-Modus (106), in dem der Hauptsensor (3) inaktiv und der Hilfssensor (4) aktiv ist.

## Revendications

1. Un réfrigérateur (1) comprenant au moins un compartiment de stockage pour stocker et réfrigérer des aliments ; une unité de détection d'odeurs (2) comprenant un capteur principal (3) et un capteur auxiliaire (4) pour détecter les odeurs dans le compartiment de stockage, et un moyen de traitement (5) pour recevoir et traiter les données des capteurs principal et auxiliaire (3, 4) ; et un écran (6) pour afficher des informations ; **caractérisé par le fait que** l'unité de détection d'odeurs (2) a un mode de fonctionnement normal pour détecter les odeurs dans le compartiment de stockage et un mode de contrôle de la sensibilité pour vérifier la sensibilité du capteur principal (3), dans lequel le capteur auxiliaire (4) est inactif dans le mode de fonctionnement normal et les deux capteurs (3, 4) sont actifs dans le mode de contrôle de la sensibilité.

2. Un réfrigérateur (1) selon la déclaration 1, dans lequel le capteur principal (3) et le capteur auxiliaire (4) sont des capteurs à oxyde métallique (MOX) réagissant en fonction du niveau de composés organiques volatils (COV) dans le compartiment de stockage.

3. Un réfrigérateur (1) selon la déclaration 1 ou 2, dans lequel le moyen de traitement (5) est conçu pour, dans le mode de vérification de la sensibilité, comparer les réactions du capteur principal (3) et du capteur auxiliaire (4) afin de déterminer un état de fonctionnement actuel du capteur principal (3).

4. Un réfrigérateur (1) selon la déclaration 3, dans lequel le moyen de traitement (5) est en outre agencé pour générer un signal pour émettre un avertissement si la différence entre les réactions du capteur principal (3) et du capteur auxiliaire (4) est supérieure à un seuil prédéterminé.

5. Un réfrigérateur (1) selon la déclaration 4, dans lequel l'avertissement comprend une indication visuelle sur l'écran (6).

6. Un réfrigérateur (1) selon la déclaration 4 ou 5, dans lequel l'avertissement comprend une invitation à faire fonctionner l'unité de détection d'odeurs (2) dans un mode de secours dans lequel le capteur principal (3) est inactif et le capteur auxiliaire est actif (4).

7. Un réfrigérateur (1) selon l'une quelconque des déclarations 4 à 6, dans lequel le moyen de traitement (5) est en outre conçu pour, si la différence entre les réactions du capteur principal (3) et du capteur auxiliaire (4) est supérieure au seuil prédéterminé, faire passer l'unité de détection (2) à un mode de secours dans lequel le capteur principal (3) est inactif et le capteur auxiliaire est actif (4).

8. Un réfrigérateur (1) selon l'une quelconque des déclarations 4 à 7, dans lequel le moyen de traitement (5) est en outre agencé pour, si la différence entre les réactions du capteur principal (3) et du capteur auxiliaire (4) est supérieure au seuil prédéterminé, bloquer le fonctionnement de l'unité de détection d'odeurs (2) en mode normal.

9. Un réfrigérateur (1) selon l'une quelconque des déclarations précédentes, dans lequel le moyen de traitement (5) est agencé pour activer/désactiver automatiquement le mode de contrôle de la sensibilité selon un horaire préprogrammé.

10. Un réfrigérateur (1) selon la déclaration 9, dans lequel l'horaire préprogrammé comprend une routine périodique.

11. Une méthode de contrôle d'un réfrigérateur (1) comprenant : au moins un compartiment de stockage pour stocker et réfrigérer des aliments ; une unité de détection d'odeurs (2) comprenant un capteur principal (3) et un capteur auxiliaire (4) pour détecter les odeurs dans le compartiment de stockage, et un moyen de traitement (5) pour recevoir et traiter les données des capteurs principal et auxiliaire (3, 4) ; et un écran (6) ; la méthode comprenant:
faire fonctionner l'unité de détection d'odeurs (2) dans un mode de fonctionnement normal (101) dans lequel le capteur principal (3) est actif et le capteur auxiliaire (4) est inactif,
déterminer si les critères de passage à un mode de contrôle de la sensibilité sont remplis (102), et si les critères ne sont pas remplis, poursuivre le fonctionnement en mode normal,
si les critères sont remplis, faire fonctionner l'unité de détection d'odeurs (2) en mode de vérification de la sensibilité (103) dans lequel le capteur principal (3) et le capteur auxiliaire (4) sont tous deux actifs,
déterminer si la différence entre les réactions du capteur principal (3) et du capteur auxiliaire (4) est supérieure à un seuil prédéterminé ; dans la négative, ramener l'unité de détection d'odeurs (2) en mode de fonctionnement normal (101), et
si ladite différence est supérieure au seuil, générer un signal d'avertissement (105).

12. Une méthode comme dans la déclaration 11, dans laquelle la méthode comprend en outre, si la différence entre les réactions du capteur principal (3) et du capteur auxiliaire (4) est supérieure au seuil prédéterminé (7), le fonctionnement de l'unité de détection d'odeurs dans un mode de sauvegarde (106) dans lequel le capteur principal (3) est inactif et le capteur auxiliaire (4) est actif.
